# EUROPEAN PATENT SPECIFICATION

(11) **EP 3 721 912 B1**
(45) Date of publication and mention of the grant of the patent: **12.07.2023**
(21) Application number: 19209505.7
(22) Date of filing: 15.11.2019
(51) Int. Cl.: A61L 9/20, A61L 9/014, A61L 9/12, B01D 53/88

(54) **DEODORIZATION DEVICE**
DESODORISIERUNGSVORRICHTUNG
DISPOSITIF DE DÉSODORISATION

(30) Priority: 09.04.2019 JP 2019073968
(43) Date of publication of application: 14.10.2020
(73) Proprietor: Kaltech Corporation, Osaka-shi, Osaka 541-0059 (JP)
(72) Inventor: HARAZONO, Toyohiro, Osaka-shi, Osaka 5410059 (JP); SOMEI, Junichi, Osaka-shi, Osaka 5410059 (JP)
(74) Representative: Horn Kleimann Waitzhofer Patentanwälte PartG mbB

(56) References cited:
- EP-A1- 1 191 951
- WO-A1-2018/008889
- CN-A- 109 023 872
- JP-A- 2018 164 508

## Description

### Technical Field

The present invention relates to a technology for deodorization devices for use in deodorization and sterilization of air with the use of a photocatalyst, activated carbon, or the like.

### Background Art

Deodorization devices are known that are intended for deodorization and sterilization of air. For example, JP-A-2014-219130 discloses a refrigerator using a photocatalyst. In the refrigerator disclosed in JP-A-2014-219130, a catalyst base supporting a photocatalyst is attached in such an orientation that the photocatalyst is exposed to the storage compartment side of a back panel constituting a cold air passage, and a light emitting diode is provided on the inner surface of an inner box constituting the cold air passage so as to emit light toward the catalyst base. With the catalyst base provided as a part of the back panel, and the photocatalyst excited by the light emitting diode provided on the inner box, the catalyst base can be sized as desired to ensure a sufficient catalyst area. This enables effective removal of odor components and germs in a large-volume storage compartment. Another advantage is that the carbon dioxide generated by the catalytic action of the photocatalyst can reduce the oxygen concentration in the storage compartment, keeping vegetables fresh for a longer time period by inhibiting the breathing of vegetables.
Further, EP 1 191 951 A1 discloses a battery powered volatile dispenser for dispensing a volatile material, the volatile dispenser including a housing having an air inlet, an air outlet, an airflow path therebetween, and a holder for a volatile reservoir for supplying a volatile to be introduced into air flowing in the airflow path. The volatile dispenser has a fan adapted to be powered by a battery, the fan being so located that it propels air through the airflow path and also is unrestrictedly visible to a user of the volatile dispenser.
WO 2018/008889 A1 discloses a deodorization module comprising: a housing having one surface including a curved surface; a suction port formed on one surface of the housing to suction external air; a fan disposed in the suction port and suctioning air; a discharge port formed on one surface of the housing to discharge the air suctioned through the fan into the outside of the housing; a photocatalytic bar disposed between the fan and the discharge port and including a through hole through which air passes; and a light source module for irradiating ultraviolet rays onto the photocatalytic bar.
CN 109 023 872 A discloses a cabinet into which clothes are placed which includes a drying care component, a sterilization component, a perfume component and a heating component.
JP 2018 - 164508 A discloses a deodorizing apparatus that includes a sheet to which a photocatalyst is added, a light source that irradiates the sheet with light, and blowing means that blows air along the front and back surfaces of the sheet. In this deodorizing apparatus, since air does not pass through the filter, pressure loss of air hardly occurs. Because of this, the rated capacity of the blower or the like can be reduced. Also, since filter clogging does not occur, the time required for maintenance can be reduced.

### Summary of Invention

### Technical Problem

An object of the present invention is to provide a deodorization device that can be used even when positioned in a variety of ways.

### Solution to Problem

This object is solved by the deodorization device defined in claim 1.

### Advantageous Effects of Invention

A deodorization device provided according to the foregoing aspect can be used even when positioned in a variety of ways.

### Brief Description of Drawings

FIG. 1 is a front perspective view showing the exterior of a photocatalytic device according to First Embodiment.
FIG. 2 is a front view showing the exterior of the photocatalytic device according to First Embodiment.
FIG. 3 is a left side view showing the exterior of the photocatalytic device according to First Embodiment.
FIG. 4 is a plan view showing the exterior of the photocatalytic device according to First Embodiment.
FIG. 5 is a bottom perspective view showing a top cabinet member according to First Embodiment.
FIG. 6 is a top perspective view showing a bottom cabinet member according to First Embodiment.
FIG. 7 is a top perspective view of the photocatalytic device according to First Embodiment with the top cabinet member of a cabinet removed.
FIG. 8 is a top perspective view of the photocatalytic device according to First Embodiment with the top cabinet member and a photocatalyst sheet removed.
FIG. 9 is a bottom perspective view of the photocatalytic device according to First Embodiment with the bottom cabinet member of the cabinet removed.
FIG. 10 is a cross sectional view at A-A in FIG. 4.
FIG. 11 is a diagram schematically representing a photocatalyst sheet according to First Embodiment.
FIG. 12 is a block diagram representing a configuration of the photocatalytic device according to First Embodiment.
FIG. 13 is a perspective view showing the photocatalytic device according to First Embodiment in upright position with the front surface at the bottom.
FIG. 14 is a perspective view showing the photocatalytic device according to First Embodiment in upright position with the left side surface at the bottom.
FIG. 15 is a plan view of a photocatalytic device according to Third Embodiment.
FIG. 16 is a reference diagram for describing a photocatalytic device according to Fourth Embodiment.
FIG. 17 shows a front view of the photocatalytic device according to Fourth Embodiment.
FIG. 18 shows a side view of the photocatalytic device according to Fourth Embodiment.

### Description of Embodiments

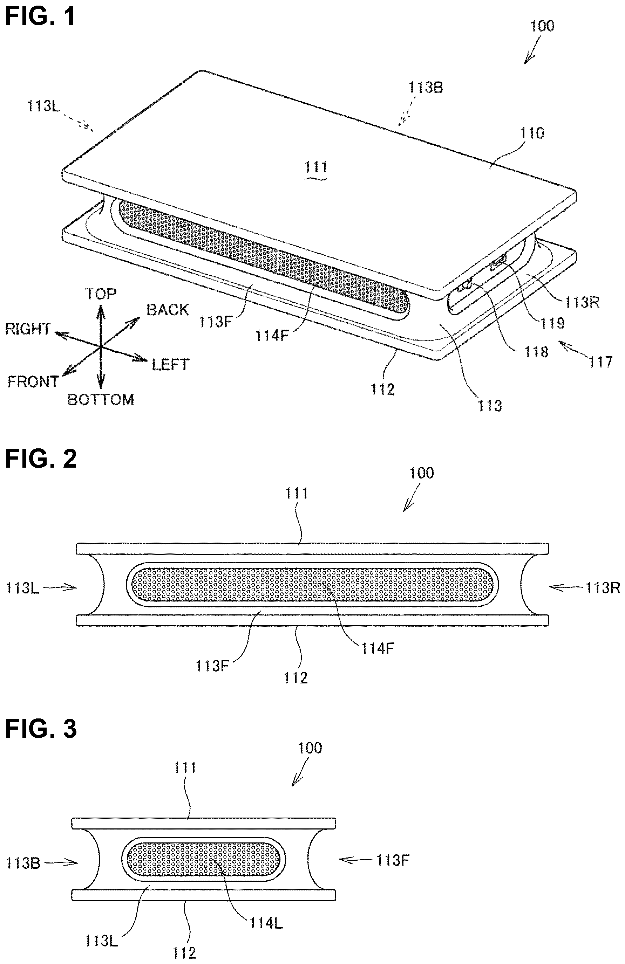

The following describes embodiments of the present invention with reference to the accompanying drawings. Specifically, a photocatalytic device 100 equipped with a photocatalyst sheet is described as an example of a deodorization device. In the following descriptions, like elements are given like reference numerals. Such like elements will be referred to by the same names, and have the same functions. Accordingly, detailed descriptions of such elements will not be repeated. In the following descriptions of the photocatalytic device 100, including the external appearance and the internal configuration of the photocatalytic device 100, the terms used to refer to directions, such as front, back, left, right, top, and bottom, are relative to the horizontally placed position of the photocatalytic device 100 as shown in FIG. 1.

### First Embodiment

### External Configuration of Photocatalytic Device 100

The external configuration of the photocatalytic device 100 according to the present embodiment is described first. Referring to FIGS. 1 to 4, the photocatalytic device 100 according to the present embodiment is covered with a cabinet 110 of a substantially rectangular shape.

The main components of the cabinet 110 include a top plate 111, a bottom plate 112, and a side member 113. The side member 113 has a right side surface 113R at which a control 117 is disposed, a front surface 113F where an inlet 114F is formed, a left side surface 113L where an outlet 114L is formed, and a back surface 113B where an inlet 114B is formed.

Specifically, in the photocatalytic device 100 according to the present embodiment, the inlet 114F at the front surface, and the outlet 114L at the left side surface are disposed at a right angle to each other. The inlet 114B at the back surface, and the outlet 114L at the left side surface are also disposed at a right angle to each other. The inlet 114F at the front surface, and the inlet 114B at the back surface are parallel to each other.

The right side surface 113R, the front surface 113F, the left side surface 113L, and the back surface 113B have a depressed shape in the surface as a whole. In other words, as viewed from the front, the right side surface 113R and the left side surface 113L have a substantially U-shape, and the front surface 113F and the back surface 113B, as viewed from the left, have a substantially U-shape. To put it in different words, substantially middle portions of the right side surface 113R, the front surface 113F, the left side surface 113L, and the back surface 113B between the top and bottom are depressed more inwardly toward the photocatalytic device 100 than the top and bottom portions.

In other words, the right side surface 113R has a groove from its front end portion to rear end portion. The front surface 113F has a groove from its left end portion to right end portion. The left side surface 113L has a groove from its front end portion to rear end portion. The back surface 113B has a groove from its left end portion to right end portion. That is, the cabinet 110 according to the present embodiment has grooves formed around the whole periphery of the side member 113.

The inlet 114F is formed in a depressed portion at a substantially middle portion of the front surface 113F between the top and bottom. The outlet 114L is formed in a depressed portion at a substantially middle portion of the left side surface 113L between the top and bottom. The inlet 114B is formed in a depressed portion at a substantially middle portion of the back surface 113B between the top and bottom. In the present embodiment, large numbers of punched holes are formed within the inlet 114F, the outlet 114L, and the inlet 114B. It is to be noted, however, that the inlet 114F, the outlet 114L, and the inlet 114B may simply have a large aperture formed therein.

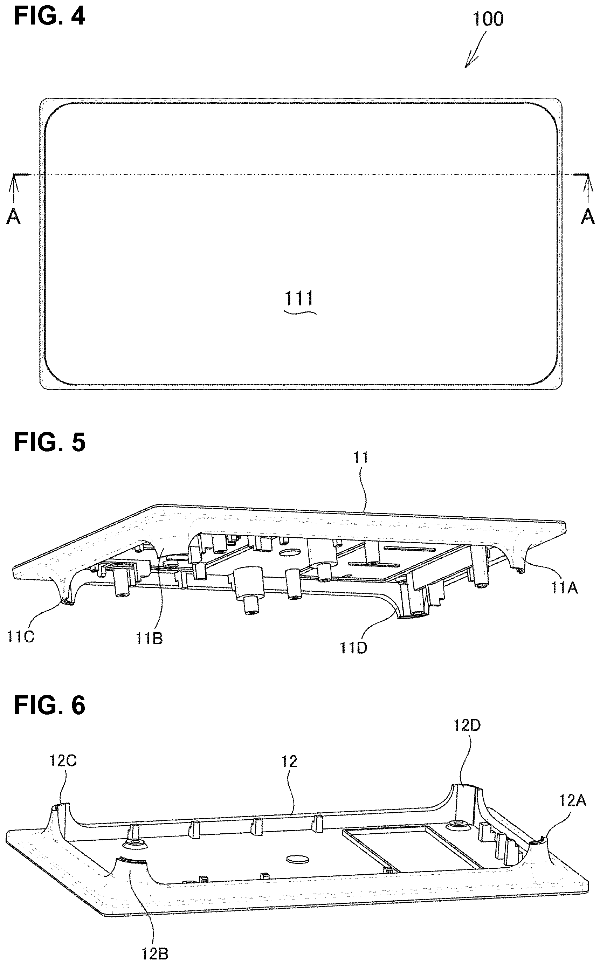

In the present embodiment, a top cabinet member 11 as shown in FIG. 5, and a bottom cabinet member 12 as shown in FIG. 6 are mated to each other to constitute the cabinet 110. A plate having punched holes for the inlets 114F and 114B, a plate having punched holes for the outlet 114L, and a plate at which a control 117 is disposed are fitted and secured between the cabinet members 11 and 12.

Specifically, the top cabinet member 11 has four downwardly extending joint members 11A, 11B, 11C, and 11D. The bottom cabinet member 12 has four upwardly extending joint members 12A, 12B, 12C, and 12D. The inlet 114F, situated at the front, is formed between the joint members 11A, 11B, 12A, and 12B. The outlet 114L, situated on the left, is formed between the joint members 11B, 11C, 12B, and 12C. The inlet 114B, situated at the back, is formed between the joint members 11C, 11D, 12C, and 12D. The control 117 is disposed between the joint members 11D, 11A, 12D, and 12A.

### Internal Configuration of Photocatalytic Device 100

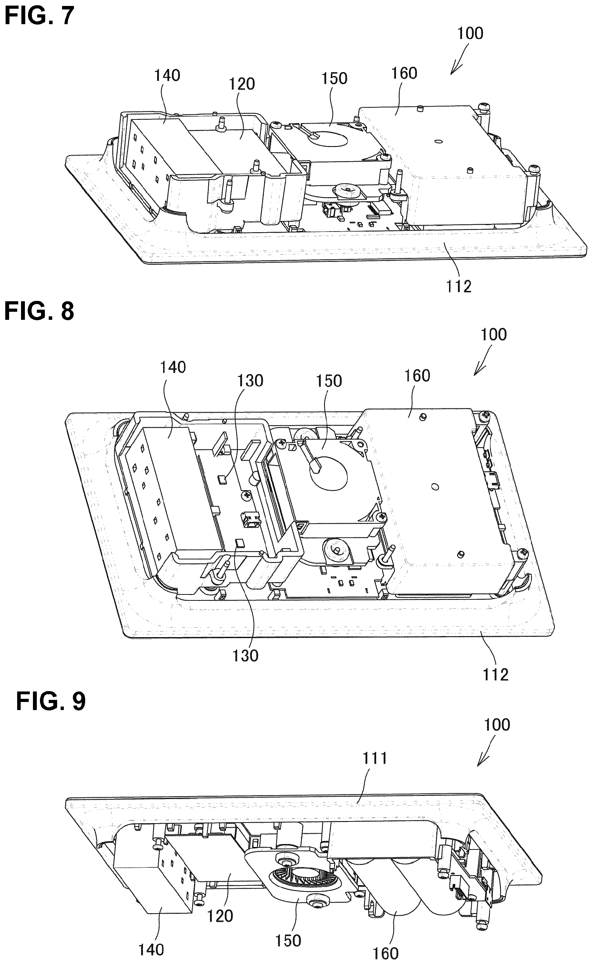

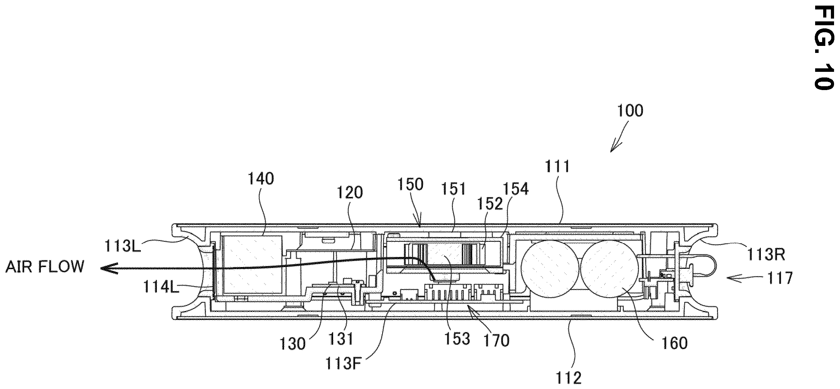

The internal configuration of the photocatalytic device 100 according to the present embodiment is described below. Referring to FIGS. 7 to 10, the main components of the photocatalytic device 100 according to the present embodiment include a photocatalyst sheet 120, LED lights 130, a deodorization filter 140, a blower fan 150, a power supply 160, a control unit 170, and the cabinet 110 housing these members.

The power supply 160 is disposed in a right portion inside the cabinet 110. The power supply 160 supplies power to the LED lights 130, the blower fan 150, and the control unit 170.

The blower fan 150 is disposed in an upper portion inside the cabinet 110, substantially in the middle between the left and right of the cabinet 110, that is, between the inlets 114F and 114B. The blower fan 150 includes a motor 151, impellers 152, a rotational shaft 153, and a case 154. The rotational shaft 153 of the impellers 152 is vertically disposed in the photocatalytic device 100. The case 154 has an open bottom surface in a portion corresponding in position to the inner side of the impellers 152. The left side of the case 154 is also opened. The top, back, right, and front surfaces of the case 154 are closed. The bottom surface of the case 154 is closed in a portion corresponding in position to the outer side of the impellers 152.

In this way, driving the motor 151 causes the air beneath the blower fan 150 to be drawn into the blower fan 150, or, more specifically, the inner side of the impellers 152, and the air is blown out of the outer side of the impellers 152, to the front and back and left and right. The blown air is directed toward the left side of the photocatalytic device 100 along the inner surfaces of the case 154 of the blower fan 150, creating an air flow toward the photocatalyst sheet 120.

The photocatalyst sheet 120 is horizontally disposed in an upper left portion of the photocatalytic device 100.

The photocatalyst sheet 120 according to the present embodiment is described below. By photoreaction, the photocatalyst sheet 120 decomposes the air oxygen and moisture adsorbed to its surface, and generates highly reactive superoxide anion (O₂⁻) and OH radical (_{·}OH). The superoxide anion (O₂⁻) and OH radical (·OH) are able to decompose odor components and remove germs, and reduce deterioration of food by decomposing ethylene gas. Examples of the photocatalyst sheet 120 include, but are not limited to, zinc oxide (ZnO), cadmium sulfide (CdS), tungsten trioxide (WO₃), and titanium dioxide (TiO₂).

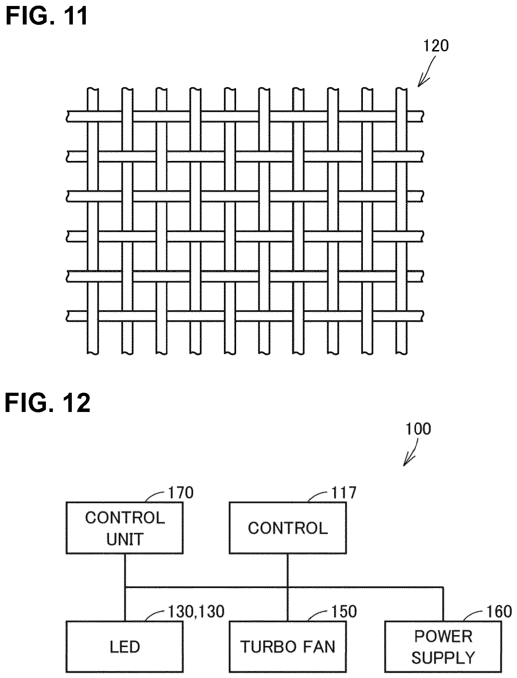

In the present embodiment, as shown in FIG. 11, the photocatalyst sheet 120 has a form of a mesh to increase its surface area.

Referring back to FIGS. 7 to 10, in the photocatalytic device 100 according to the present embodiment, the photocatalyst sheet 120 is disposed substantially parallel to the air flow from the blower fan 150. That is, in the present embodiment, the photocatalyst sheet 120 is disposed parallel to the top plate 111 and the bottom plate 112. In other words, in the present embodiment, the photocatalyst sheet 120 is disposed orthogonal to the outlet 114L and the inlets 114F and 114B.

More specifically, the cabinet 110 of the photocatalytic device 100 has the longest dimension side to side, the second longest dimension front to back, and the shortest dimension up and down. The photocatalyst sheet 120 is disposed in such an orientation that its normal line coincides with the up-down direction, which corresponds to the shortest dimension of the cabinet 110 of the photocatalytic device 100. In other words, the photocatalyst sheet 120 is disposed in such an orientation that the up-down direction of the photocatalytic device 100 is perpendicular to the photocatalyst sheet 120.

The LED lights 130 on a substrate 131 are disposed below the photocatalyst sheet 120. In the present embodiment, two LED lights 130 are provided, one in a position facing a front portion of the photocatalyst sheet 120, and one in a position facing a rear portion of the photocatalyst sheet 120.

The photocatalyst sheet 120 is held a distance away from the inner side of the top plate 111 of the cabinet 110, or from a surface that is present above the photocatalyst sheet 120. That is, a space is created above the photocatalyst sheet 120. In this way, more light can reach the top surface of the photocatalyst sheet 120 from the LED lights 130, and the photoreaction can produce stronger decomposition.

When the inner side of the top plate 111 of the cabinet 110 has a whiter color or a color that more readily reflects light, even more light can reach the top surface of the photocatalyst sheet 120 from the LED lights 130, and the photoreaction can produce even stronger decomposition. It is possible to even more increase the quantity of the light that reaches the top surface of the photocatalyst sheet 120 from the LED lights 130, and provide even stronger decomposition by photoreaction when a reflecting plate is disposed on the inner side of the top plate 111 of the cabinet 110, or above the photocatalyst sheet 120, to reflect light from the LED lights 130.

The deodorization filter 140 for deodorization is disposed on the right side of the photocatalyst sheet 120. In other words, the deodorization filter 140 is disposed between the photocatalyst sheet 120 and the outlet 114L. The deodorization filter 140 is, for example, a filter with deodorizing capabilities, such as an oxidation catalyst, a low-temperature catalyst, and activated carbon.

### Control Configuration of Photocatalytic Device 100

The photocatalytic device 100 according to the present embodiment is described below with regard to its control configuration. As shown in FIG. 12, the main components of the photocatalytic device 100 include the control unit 170, the control 117, the LED lights 130, the blower fan 150, and the power supply 160.

In response to power being turned on via the control 117, the control unit 170 uses the power from the power supply 160 to drive the blower fan 150, and turn on the front and back LED lights 130. In response, the air drawn in by the blower fan 150 through the inlets 114F and 114B becomes sterilized and deodorized by the photocatalyst sheet 120, and the deodorized air through the deodorization filter 140 discharges through the outlet 114L.

As shown in FIG. 3, the control 117 includes an ON/OFF switch 118, and a USB interface 119 for charging, among others.

### Review of Photocatalytic Device 100 According to First Embodiment

As described above, in the present embodiment, the inlet 114F at the front surface and the outlet 114L at the left side surface are disposed at a right angle to each other, and the inlet 114B at the back surface and the outlet 114L at the left side surface are disposed also at a right angle to each other. Because the photocatalyst sheet 120 is horizontally disposed, the photocatalytic device 100 can be made compact.

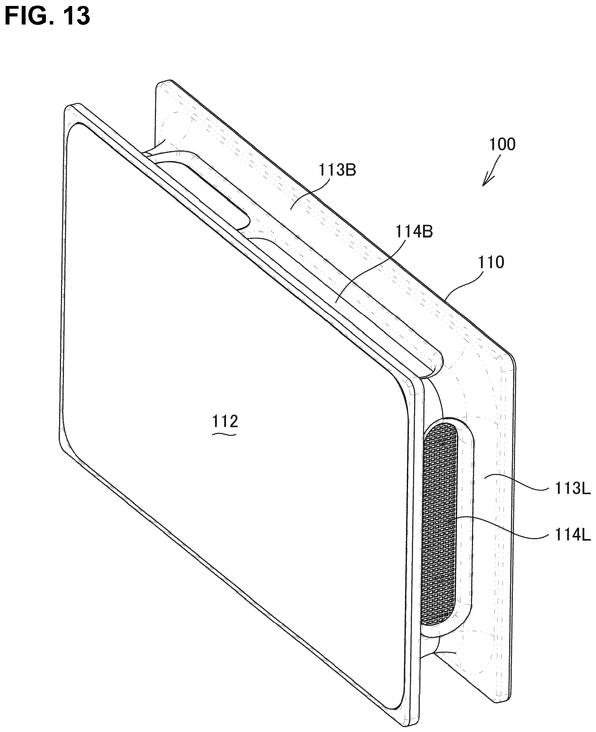

Because the cabinet 110 has grooves formed around the whole periphery of its side plate 113, air can be easily drawn in from the inlet 114F through the groove formed in the front surface 113F, even when the photocatalytic device 100 is used in upright position with the front surface 113F at the bottom, as illustrated in FIG. 13. Likewise, air can be easily drawn in from the inlet 114B through the groove of the back surface 113B even when the photocatalytic device 100 is used in upright position with the back surface 113B at the bottom.

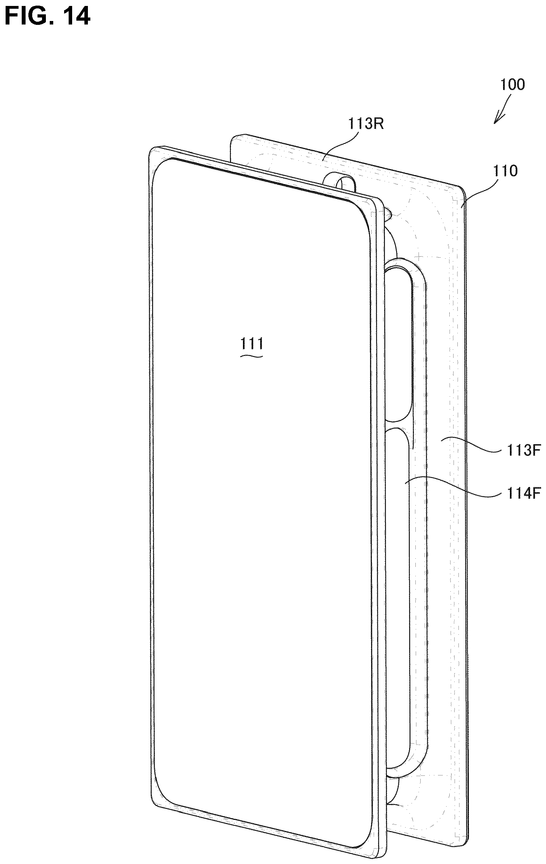

Air can also easily discharge from the outlet 114L through the groove of the left side surface 113L, even when the photocatalytic device 100 is used in upright position with the left side surface 113L at the bottom, as illustrated in FIG. 14.

Even in situations where objects are placed beside the photocatalytic device 100 horizontally placed as illustrated in FIG. 1, it is possible to ensure that air is easily drawn in from the inlet 114F through the groove of the front surface 113F, and from the inlet 114B through the groove of the back surface 113B, and easily discharges from the outlet 114L through the groove of left side surface 113L.

### Second Embodiment

In the foregoing embodiment, the right side surface 113R and the left side surface 113L have grooves all the way from the front end to the rear end, and the grooves are as wide as to reach the top end and the bottom end. That is, nearly all portions of the right side surface 113R and the left side surface 113L are forming the grooves. However, the left side surface 113L may have a groove reaching the outlet 114L from either the front end or rear end, provided that the groove is in communication with the outlet 114L, and the groove may be formed only in a portion of the surface between the top and bottom, provided that the groove is in communication with the outlet 114L.

Similarly, the front surface 113F and the back surface 113B have grooves all the way from the right end to the left end, and the grooves are as wide as to reach the top end and the bottom end. That is, nearly all portions of the front surface 113F and the back surface 113B are forming the grooves. However, the front surface 113F may have a groove reaching the inlet 114F from either the right end or left end, provided that the groove is in communication with the inlet 114F. The back surface 113B may have a groove reaching the inlet 114B from either the right end or left end, provided that the groove is in communication with the inlet 114B. In the front surface 113F and the back surface 113B, the groove may be formed only in a portion of the surface between the top and bottom, provided that the groove is in communication with the inlet 114F or 114B.

### Third Embodiment

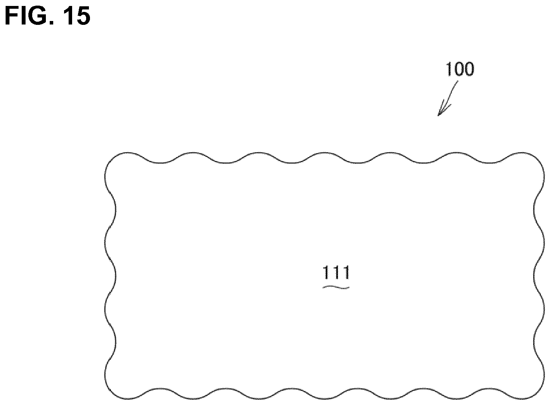

The grooves are not limited to the grooves formed front to back or side to side, and may be vertical grooves, or irregularities that can be visually recognized as being depressed in plan view, as illustrated in FIG. 15.

### Fourth Embodiment

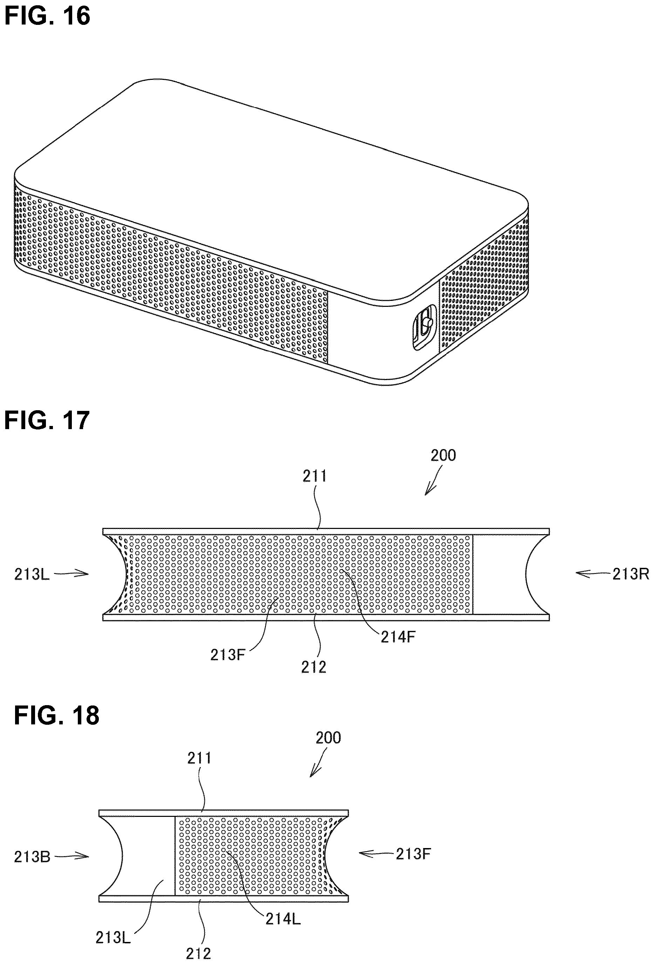

The groove configurations on the exterior of the cabinet 110 according to the foregoing embodiments, and the internal configuration of the cabinet 110 according to the foregoing embodiment are applicable to photocatalytic devices 100 of other configurations. For example, the groove configurations on the exterior of the cabinet 110 of the photocatalytic device 100 described above, and the internal configuration of the cabinet 110 are applicable to the photocatalytic device shown in FIG. 16.

That is, the right side surface, the front surface, the left side surface, and the back surface may have a depressed shape in the surface as a whole. More specifically, as shown in FIG. 17, the right side surface 213R and the left side surface 213L may have a substantially U-shape as viewed from the front, and, as shown in FIG. 18, the front surface 213F and the back surface 213B may have a substantially U-shape as viewed from the left. To put it in different words, substantially middle portions of the right side surface 213R, the front surface 213F, the left side surface 213L, and the back surface 213B between the top and bottom are depressed more inwardly toward the photocatalytic device 200 than the top and bottom ends.

The groove configurations on the exterior of the cabinet 110 according to the foregoing embodiments are also applicable to photocatalytic devices that are not substantially rectangular in shape. For example, the groove configurations are applicable to photocatalytic devices of triangular and other polygonal shapes, and to photocatalytic devices of elliptical and circular shapes as viewed in plan.

### Fifth Embodiment

The descriptions of the foregoing embodiments are based on the photocatalytic device 100 having a single photocatalyst sheet 120. However, the technology of the foregoing embodiments is also applicable to a photocatalytic device having a plurality of photocatalyst sheets 120. For example, two photocatalyst sheets 120 may be horizontally disposed so as to face each other on the downstream side of the blower fan 150, parallel to the air flow from the blower fan 150, that is, orthogonal to the outlet and the inlets. In this case, the LED lights 130 may be disposed between the two photocatalyst sheets 120, or on the front, back, right, or left side of the photocatalyst sheets 120.

### Sixth Embodiment

In the foregoing embodiments, the photocatalytic device 100 having the photocatalyst sheet 120 was described as an example of the deodorization device. However, the technology of the foregoing embodiments is also applicable to photocatalytic devices having photocatalysts that are not sheet-like in shape, and to deodorization devices that are not equipped with a photocatalyst but are equipped with the deodorization filter 140 and other deodorizing members.

### Review

In the foregoing embodiments, a deodorization device is provided that includes: a cabinet having an inlet and an outlet; a deodorizing member disposed inside the cabinet; a light disposed inside the cabinet; and a fan disposed inside the cabinet. The deodorization device has a depression formed in a cabinet surface where the inlet is formed, and the depression reaches the inlet from an end portion of the cabinet surface.

In the foregoing embodiments, a deodorization device is provided that includes: a cabinet having an inlet and an outlet; a photocatalyst member disposed inside the cabinet; a light disposed inside the cabinet; and a fan disposed inside the cabinet. The deodorization device has a depression formed in a cabinet surface where the outlet is formed, and the depression reaches the outlet from an end portion of the cabinet surface.

Preferably, the cabinet is substantially rectangular in shape.

Preferably, the depression is formed across the surface.

Preferably, the depression runs around side surfaces of the cabinet.

The embodiments disclosed herein are to be considered in all aspects only as illustrative and not restrictive. The scope of the present invention is to be determined by the scope of the appended claims, not by the foregoing descriptions, and the invention is intended to cover all modifications falling within the equivalent meaning and scope of the claims set forth below.

### Reference Signs List

11: Top cabinet member
11A: Joint member
11B: Joint member
11C: Joint member
11D: Joint member
12: Cabinet member
12A: Joint member
12B: Joint member
12C: Joint member
12D: Joint member
100: Photocatalytic device
110: Bottom side of cabinet
111: Top plate
112: Bottom plate
113: Side member
113B: Back surface
113F: Front surface
113L: Left side surface
113R: Right side surface
114B: Inlet
114F: Inlet
114L: Outlet
117: Control
118: ON/OFF switch
119: USB interface
120: Photocatalyst sheet
130: LED light
131: Substrate
140: Deodorization filter
150: Blower fan
151: Motor
152: Impellers
153: Rotational shaft
154: Case
160: Power supply
170: Control unit
200: Photocatalytic device
213B: Back surface
213F: Front surface
213L: Left side surface
213R: Right side surface

## Claims

1. A deodorization device (100) comprising:
a cabinet (110) having an inlet (114F, 114B) and an outlet (114L);
a deodorizing member (120, 140) disposed inside the cabinet (110);
a light (130) disposed inside the cabinet (110); and
a fan (150) disposed inside the cabinet (110),
wherein the cabinet (110) includes a top plate (111), a bottom plate (112) and a side member (113) arranged between the top plate (111) and the bottom plate (112), the side member (113) extending around a periphery of the cabinet (110);
**characterized in that**:
the deodorization device (100) has a depression formed in a cabinet surface where the inlet (114F, 114B) and the outlet (114L) are formed,
the depression reaches the inlet (114F, 114B) and the outlet (114L) from an end portion of the cabinet surface, and
the depression is a groove formed around a whole periphery of the side member (113) of the cabinet (110).

2. The deodorization device (100) according to claim 1, wherein the cabinet (110) is substantially rectangular in shape.

3. The deodorization device (100) according to claim 1 or 2, wherein the depression is formed across the surface.

4. The deodorization device (100) according to claim 3, wherein the depression has a U-shape.

## Patentansprüche

1. Deodorisierungsvorrichtung (100), aufweisend:
ein Gehäuse (110) mit einem Einlass (114F, 114B) und einem Auslass (114L);
ein Deodorisierungselement (120, 140), das im Inneren des Gehäuses (110) angeordnet ist;
ein Licht (130), das im Inneren des Gehäuses (110) angeordnet ist; und
ein im Gehäuse (110) angeordnetes Gebläse (150),
wobei das Gehäuse (110) eine obere Platte (111), eine untere Platte (112) und ein zwischen der oberen Platte (111) und der unteren Platte (112) angeordnetes Seitenelement (113) aufweist, wobei das Seitenelement (113) sich um den Umfang des Gehäuses (110) erstreckt.
**dadurch gekennzeichnet, dass**:
die Deodorisierungsvorrichtung (100) eine Vertiefung aufweist, die in einer Gehäuseoberfläche ausgebildet ist, in der der Einlass (114F, 114B) und der Auslass (114L) ausgebildet sind,
die Vertiefung bis von einem Endabschnitt der Gehäuseoberfläche bis zum Einlass (114F, 114B) und zum Auslass (114L) reicht, und
die Vertiefung eine Nut ist, die um den gesamten Umfang des Seitenelements (113) des Gehäuses (110) ausgebildet ist.

2. Deodorisierungsvorrichtung (100) nach Anspruch 1, wobei das Gehäuse (100) eine im Wesentlichen rechteckige Form aufweist.

3. Deodorisierungsvorrichtung (100) nach Anspruch 1 oder 2, wobei die Vertiefung flächendeckend ausgebildet ist.

4. Deodorisierungsvorrichtung (100) nach Anspruch 3, wobei die Vertiefung U-förmig ist.

## Revendications

1. Dispositif de désodorisation (100) comprenant :
un boîtier (110) ayant une entrée (114F, 114B) et une sortie (114L) ;
un élément désodorisant (120, 140) disposé à l'intérieur du boîtier (110) ;
un organe d'éclairage (130) disposé à l'intérieur du boîtier (110) ; et
un ventilateur (150) disposé à l'intérieur du boîtier (110),
dans lequel le boîtier (110) comprend une plaque supérieure (111), une plaque inférieure (112) et un élément latéral (113) agencé entre la plaque supérieure (111) et la plaque inférieure (112), l'élément latéral (113) s'étendant autour d'une périphérie du boîtier (110);
**caractérisé en ce que** :
le dispositif de désodorisation (100) a une dépression formée dans une surface de boîtier où l'entrée (114F, 114B) et la sortie (114L) sont formées,
la dépression atteint l'entrée (114F, 114B) et la sortie (114L) depuis une partie d'extrémité de la surface du boîtier, et
la dépression est une gorge ménagée sur tout le pourtour du longeron (113) du carter (110).

2. Dispositif de désodorisation (100) selon la revendication 1, dans lequel le boîtier (110) est de forme sensiblement rectangulaire.

3. Dispositif de désodorisation (100) selon la revendication 1 ou 2, dans lequel la dépression est formée sur toute la surface.

4. Dispositif de désodorisation (100) selon la revendication 3, dans lequel la dépression a une forme en U.
